Europäisches Patentamt

European Patent Office

Office européen des brevets

(19)

(11) Veröffentlichungsnummer: **0 380 140**

**A2**

(12) # EUROPÄISCHE PATENTANMELDUNG

(21) Anmeldenummer: 90103035.3

(22) Anmeldetag: 09.04.87

(51) Int. Cl.5: **A61M 1/28, H05B 3/30, H01C 7/02**

Diese Anmeldung is am 16 - 02 - 1990 als Teilanmeldung zu der unter INID-Kode 60 erwähnten Anmeldung eingereicht worden.

(30) Priorität: **11.04.86 HU 153686**

(43) Veröffentlichungstag der Anmeldung:
**01.08.90 Patentblatt 90/31**

(60) Veröffentlichungsnummer der früheren
Anmeldung nach Art. 76 EPÜ: **0 242 724**

(84) Benannte Vertragsstaaten:
**AT BE CH DE ES FR GB IT LI NL SE**

(71) Anmelder: **ROLITRON Müszaki-Fejlesztö
Kisszövetkezet
Veréb u.3
H-1124 Budapest(HU)**

(72) Erfinder: **Kurucz, Jósef
Kisfaludy u.5
H-1082 Budapest(HU)**

(74) Vertreter: **Patentanwälte Leinweber &
Zimmermann
Rosental 7/II Aufg.
D-8000 München 2(DE)**

(54) **Verfahren und Vorrichtung zur Einführung einer Flüssigkeit in menschlichen oder tierischen Organismen sowie Verfahren und Heizmittel für Temperaturregelung.**

(57) Die Erfindung betrifft ein Heizmittel (59) zur Regelung der Temperatur von strömenden Medien, das mindestens zwei erwärmende Seitenwände (28,128) und zwischen diesen einen Kanal (47) enthält, wobei jede Seitenwand (28,128) ein erwärmendes Widerstandsnetz und einen Temperaturfühler aufweist. Das Widerstandsnetz ist aus einem an der dem zu erwärmenden Medium abgewandten Seite einer elektrisch isolierenden Tragplatte (1,101) ausgebildeten Heizfoliemuster (2,102) ausgebildet. An der anderen Seite der Tragplatte (1,101) ist ein einen temperaturfühlenden Widerstand bildendes Fühlfoliemuster (3, 103) ausgebildet, welches mit einer Isolierschicht (15,115) bedeckt ist.

Fig.1

Fig. 10

EP 0 380 140 A2

# VERFAHREN UND VORRICHTUNG ZUR EINFÜHRUNG EINER FLÜSSIGKEIT IN MENSCHLICHEN ODER TIERISCHEN ORGANISMEN, SOWIE VERFAHREN UND HEIZMITTEL FÜR TEMPERATURREGELUNG

## TECHNISCHES GEBIET

Die Erfindung betrifft ein Verfahren und eine Vorrichtung zur Einführung einer physiologischen Flüssigkeit in menschlichen oder tierischen Organismen, sowie ein dazu geeignetes Verfahren und Heizmittel zur Regelung der Temperatur von strömenden Medien.

## STAND DER TECHNIK

Zur Regelung der Temperatur von strömenden Medien ist eine Lösung bekannt, wobei die Temperatur des aus einem Heizmittel austretenden Mediums gemessen und die Heizung des Heizmittels mit einem rückgekoppelten Regelungskreis derart geregelt ist, dass das aus dem Heizmittel austretende Medium eine vorgeschriebene Temperatur aufweist. Bei dieser Lösung folgt der Regelungskreis die Änderung der Strömungsmenge oder der Temperatur des in das Heizmittel eintretender Mediums nur verhältnismässig langsam. Folgenderweise ist diese Regelung, besonders wenn die Temperatur kleinerer Mediumsmenge geregelt werden soll, nicht genug schnell.

Bei einer anderen bekannten Lösung ist das strömende Medium durch ein auf die vorgeschriebene Temperatur eingestelltes Heizmittel von grosser Wärmekapazität durchgelassen. Diese Lösung ist z.B. bei der Einführung physiologischer Flüssigkeiten in den merschlichen Organismus verwendet, wobei die Sicherheitsanforderungen nicht zulassen, dass das Heizmittel eine höhere Temperatur als die vorgeschriebene Temperatur der Flüssigkeit hat. Bei einer bekannten Methode die einzuführende physiologische Flüssigkeit wird durch eine Schlauchspule geführt, die in einem thermostatisierten Wasserbad ist.

Die EP-A-112 104 betrifft eine Vorrichtung zur Peritonealdialyse, bei welcher die Spülflüssigkeit durch einen, zwischen zwei Heizplatten von grosser Wärmekapazität angeordneten flachen Beutel in einen Fallbehälter gepumpt und von diesem durch natürliches Gefälle durch denselben Beutel in die Bauchhöhle des Patienten geleitet wird. Die Heizplatten sind ständig auf Körpertemperatur gehalten. Diese Temperaturregelung beruht auch darauf, dass das Medium, wenn es durch ein Heizmittel von grosser Wärmekapazität genügend langsam durchfliesst, bei dem Austritt die vorgeschriebene Temperatur aufweisen wird. Dieses Regelungsverfahren kann nur bei kleineren Strömungsgeschwindigkeiten verwendet werden, es nimmt die Änderungen der Temperatur der einfliessenden Flüssigkeit nicht in Betracht, erfordert ein verhältnismässig umfangreiches Heizmittel mit grossem Energieverbrauch und ist wegen seiner Trägheit bei einer Folgeregelung der Temperatur weniger anwendbar.

## OFFENBARUNG DER ERFINDUNG

Der Erfindung liegt die Aufgabe zugrunde ein Verfahren und eine Vorrichtung zu schaffen, welche ermöglichen, die Behandlungzeit bei Einführung vom physiologischen Flüssigkeiten zu verkürzen. Zu diesem Zweck sind auch ein Verfahren zur Temperaturregelung und ein Heizmittel geschaffen worden, mit welcher die Temperatur eines strömenden Mediums mit kleinerem Energieverbrauch, schnell und mit grosser Genauigkeit geregelt werden kann.

Die Erfindung bezieht sich einerseits auf ein Verfahren zur Einführung einer physiologischen Flüssigkeit bei einer vorgeschriebenen Temperatur in menschlichen oder tierischen Organismen, wobei die Flüssigkeit durch ein geregeltes Heizmittel in einen Fallbehälter gepumpt und dann aus dem Fallbehälter durch dasselbe Heizmittel in den Organismus durch natür liches Gefälle eingeleitet wird. Nach der Erfindung, während des Pumpens in den Fallbehälter wird die Temperatur der in das Heizmittel eintretenden Flüssigkeit gemessen, und aufgrund dessen wird eine im wesentlichen die vorgeschriebene Temperatur der aus dem Heizmittel austretenden Flüssigkeit ergebende Temperatur oder ein entsprechender Wärmefluss im Heizmittel eingestellt, und während der Einleitung durch natürliches Gefälle wird die Temperatur des Heizmittels auf die vorgeschriebene Temperatur der Flüssigkeit geregelt.

Der Erfindung nach handelt es sich um zwei voneinander abweichende Temperaturregelungen bei dem Pumpen in den Fallbehälter und bei der Einleitung in den Organismus. Im letzteren Falle ist die Temperatur des Heizmittels - unter Berücksichtigung der Sicherheitsanforderungen - zumindenst annähernd auf die vorgeschriebene Temperatur der Flüssigkeit eingestellt. Im Laufe des Pumpens in den Fallbehälter aber ist eine schnellere, geregelte Heizung der eintretenden Flüssigkeit mit dem Heizmittel durchgeführt. Dies ist so erreicht, dass die Temperatur der eintretenden Flüssigkeit gemessen wird, und aufgrund dessen - in Kenntnis der Parameter der Heizmittels, sowie des Massenstroms und spezifischer Wärme der Flüssigkeit - im Heizmittel die Temperatur oder der Wärmestrom so eingestellt wird, dass die Temperatur der aus dem Heizmittel austretenden Flüssigkeit zumindenst annähernd an dem vorgeschriebenen Wert sei. Bei dem Pumpen genügt es, wenn die Temperatur der in den Fallbehälter eintretenden Flüssigkeit nur annähernd der vorgeschriebenen Temperatur entspricht, da während der Einleitung in den Organismus die Flüssigkeit noch einmal durch das auf die vorgeschriebene Temperatur geregelte Heizmittel durchfliesst.

Gegenstand der Erfindung ist ferner eine Vorrichtung zur Einführung einer physiologischen Flüssigkeit in menschlichen oder tierischen Organismen bei einer vorgeschriebenen Temperatur, welche eine, die physiologische Flüssigkeit durch ein mit einem Regelungssystem versehenes Heizmittel in einen Fallbehälter befördernde Pumpe und eine, die Flüssigkeit aus dem Fallbehälter durch dasselbe Heizmittel in den Organismus einführende Leitung hat. Der Erfindung nach enthält das Regelungssystem des Heizmittels einen, die Temperatur oder den Wärmefluss in Heizmittel einstellenden Regelungskreis und für diesen Regelungskreis eine, während der Beförderung in den Fallbehälter einen, zur gemessenen Temperatur der in das Heizmittel eintretenden Flüssigkeit proportional veränderlichen Sollwert, während der Einleitung in den Organismus aber einen, das Heizmittel auf der vorgeschriebenen Temperatur der Flüssigkeit haltenden konstanten Sollwert herstellende Einheit, und ein Eingang dieser Einheit ist dem Ausgang eines, die Temperatur der in das Heizmittel eintretenden Flüssigkeit messenden ersten Signalgebers angeschlossen.

In einer Ausführungsform der Vorrichtung hat die den Sollwert herstellende Einheit auch einen anderen Eingang, der dem Ausgang eines die Temperatur der aus dem Heizmittel austretenden Flüssigkeit messenden zweiten Signalgebers angeschlossen ist, und ein Mittel, das ein der Differenz zwischen der vorgeschriebenen Temperatur der Flüssigkeit und der gemessenen Temperatur der austretenden Flüssigkeit entsprechendes Signal auf den veränderlichen Sollwert superponiert.

Die Erfindung bezieht sich andererseits auf ein Verfahren zur Regelung der Temperatur von strömenden Medien, wobei die Temperatur des strömenden Mediums gemessen und dementsprechend ein das strömende Medium erwärmende Heizmittel derart geregelt wird, dass das aus dem Heizmittel austretende Medium stets eine vorgeschriebene Temperatur aufweist. Der Erfindung nach wird die Temperatur des in das Heizmittel eintretenden Mediums gemessen und aufgrund der gemessenen Temperatur, der spezifischen Wärme, der Massenströmung und der vorgeschriebenen Temperatur des austretenden Mediums eine in wesentlichen die vorgeschriebene Temperatur des austretenden Mediums ergebende Temperatur oder ein entsprechender Wärmefluss im Heizmittel eingestellt, gleichzeitig wird auch die Temperatur des austretenden Mediums gemessen und die im Heizmittel eingestellte Temperatur oder der entsprechende Wärmefluss entsprechend der Differenz zwischen der vorgeschriebenen Temperatur und der gemessenen Temperatur des austretenden Mediums geändert.

Bei diesem Verfahren wird die Temperatur des in das Heizmittel eintretenden Mediums fortlaufend gemessen, und im Heizmittel wird eine solche Temperatur oder ein solcher Wärmefluss eingestellt, welche(r) bei der gemessenen Eintrittstemperatur die vorgeschriebene Austrittstemperatur ergibt. Dies ist selbstverständlich nur für ein Medium von gegebener spezifischer Wärme und vom gegebenen Massenstrom gültig. Wenn der Massenstrom sich bedeutend ändern kann, soll auch der Massenstrom vor der Messung der Temperature des eintretenden Mediums gemessen und die Einstellung unter dessen Berücksichtigung durchgeführt werden. Im Falle aber, wenn das strömende Medium mit Hilfe einer Pumpe von konstanter Förderleistung mit ständiger Geschwindigkeit geströmt wird, kann der Massenstrom mit einer guten Näherung als konstant angenommen werden. Diese steuertechnische Einstellung des Heizmittels(ohne Rückkopplung)wird, der Erfindung nach, entsprechend der Differenz zwischen der vorgeschriebenen Temperatur und der gemessenen Temperatur des austretenden Mediums geändert. Diese ständige Änderung ist vom Regelungscharakter (mit Rückkopplung) und sichert mit grosser Genauigkeit die vorgeschriebene Temperatur des austretenden Mediums. Die Lösung ist sowohl zu wertbehaltenden, als auch zu folgenden Temperaturregelungen geeignet. Bei einem verhältnismässig stabilen Massenstrom ist es vorteilhaft die Änderung der im Heizmittel ein gestellte Temperatur oder des entsprechenden Wärmeflusses auf ±30 %, gegebenenfalls auf ±10 % zu begrenzen um zu vermeiden, dass im Regelungssystem zu grosse Schwingungen bei dem Anlauf oder im Falle von plötzlichen Änderungen der Temperatur des eintretenden Mediums auftreten. Das Verfahren nach der Erfindung ermöglicht eine schnelle und gleichzeitig genaue Temperaturregelung.

Bei einer Ausführungsform erfolgt die Einstellung der Temperatur oder des Wärmeflusses im Heizmittel derart, dass die Heizung des Heizmittels aufgrund der im Heizmittel gemessenen Temperatur oder des im Heizmittel gemessenen Wärmeflusses, mit Hilfe eines der Temperatur des eintretenden und austretenden Mediums entsprechenden Sollwerts geregelt wird.

Gegenstand der Erfindung ist ferner ein, zu den obigen Verfahren und der obigen Vorrichtung geeignetes Heizmittel, welches mindestens zwei erwärmende Seitenwände und zwischen denen einen Kanal für das Medium enthält, wobei jede Seitenwand mindestens ein erwärmendes Widerstandsnetz aus leitungsfähigem Material und mindestens einen Temperaturfühler zur Messung der Temperatur der Seitenwand aufweist. Der Erfindung nach ist das erwärmendes Widerstandsnetz aus einem, an der dem zu erwärmenden Medium gegenüberliegenden Seite einer elektrisch isolierenden Tragplatte ausgestalteten Heizfoliemuster gebildet, und an der, nach dem zu erwärmenden Medium liegenden Seite der Tragplatte ist ein, einen temperaturfühlenden Widerstand bildendes, aus leitungsfähigem Material hergestelltes Fühlfoliemuster ausgestaltet, welches Fühlfoliemuster mit einer Isolierschicht bedeckt ist. Es ist besonders vorteilhaft, wenn das zu erwärmende Medium inhomogen bzw. eine schlechte Wärmeleitfähigkeit aufweist, an der Isolierschicht eine Temperaturausgleichschicht aus einem Material von guter Wärmeleitfähigkeit, z.B. eine Kupfer- oder Aluminiumplatte mit einer Dicke von kleiner als 2 mm anzuordnen.

Das Heizmittel nach der Erfindung ermöglicht eine sehr schnelle und genaue Temperaturregelung von strömenden Medien, infolge der kleinen Wärmeträgheit des Heizmittels und der guten Messbarkeit seiner Temperatur.Das zu erwärmende Medium kann flüssig oder gasförmig sein.

Die Erfindung wird im folgenden anhand der, in den Zeichnungen veranschaulichten Ausführungsformen beschrieben. Hierbei zeigt:

## KURZE BESCHREIBUNG DER ZEICHNUNGEN

Fig. 1 einen schematischen Anordnungsplan einer die physiologische Flüssigkeit in den menschlichen Organismus bei gewünschten Temperatur einführenden Vorrichtung nach der Erfindung,

Fig. 2 ein Blockschema eines Temperaturregelungssystems der Vorrichtung gemäss Fig. 1,

Fig. 3 ein Blockschema einer anderen vorteilhafter Ausführungsform des Temperaturregelungssystems der Vorrichtung gemäss Fig. 1,

Fig. 4 ein Blockschema einer weiteren Ausführungsform des Temperaturregelungssystems der Vorrichtung gemäss Fig. 1,

Fig. 5 eine Seite einer Tragplatte mit einem Fühlfoliemuster in einer Seitenwand des Heizmittels nach der Erfindung,

Fig. 6 die andere Seite der Tragplatte mit einem Heizfoliemuster,

Fig. 7 einen Schnitt entlang der Linie A-A in Fig. 6,

Fig. 8 einen Detailschnitt entlang der Linie B-B in Fig. 7,

Fig. 9 eine Seite der Seitenwand gemäss Fig. 6 mit einem, den Flüssigkeitskanal zwischen den Seitenwänden bildenden Beutel,

Fig. 10 einen schematischen Detailschnitt des Heizmittels nach der Erfindung,

Fig. 11 ein, die Temperaturverhältnisse im Heizmittel gemäss Fig. 10 darstellende Diagramm,

Fig. 12 eine zerlegte Darstellung der einen Seitenwand eines als Beispiel dargestellten Heizmittels und

Fig. 13 eine zerlegte Darstellung der anderen Seiten wand des Heizmittels gemäss Fig. 12.

## AUSFÜHRUNGSFORMEN DER ERFINDUNG

In den Zeichnungen sind die identischen Elemente bzw. die Elemente von identischer Funktion mit gleichen Bezugszeichen bezeichnet.

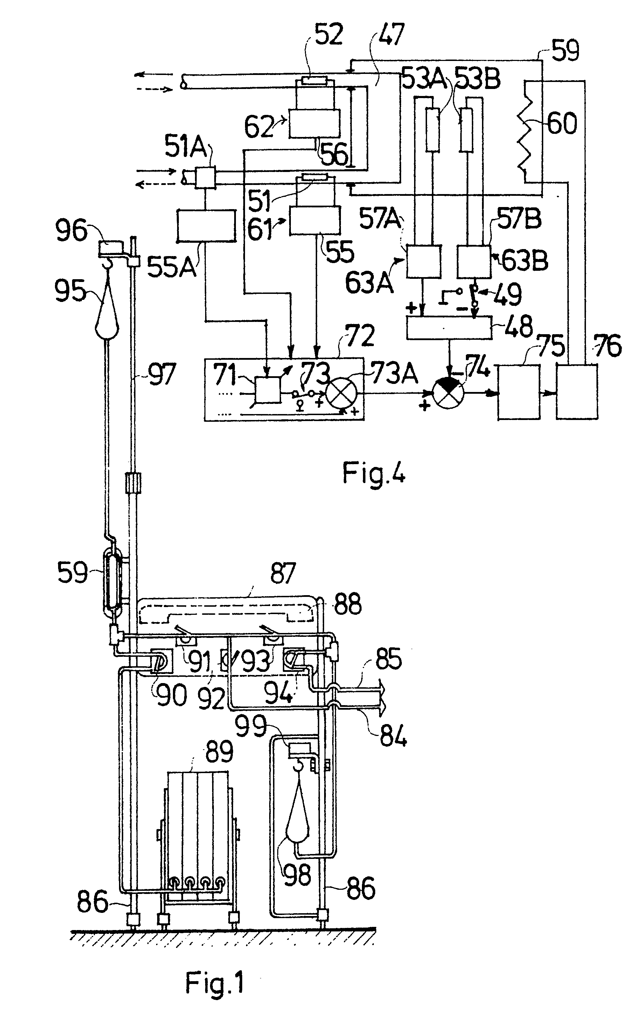

In Fig. 1 ist eine die Verwendung der erfindungsgemässen Vorrichtung darstellende Peritonealdialysevorrichtung zu sehen, welche ein erfindungsgemässes Heizmittel 59 aufweist. Die Vorrichtung hat eine am Traggestelle 86 befestigte Steuerungseinheit 87, an welcher eine Bedienungs- und Anzeigeeinheit 88 angeordnet ist. Zum Traggestelle 86 ist auch eine Haltestange 97 befestigt, an welcher ein Fallbehälter 95 durch Zwischenschaltung eines Kraftmessers 96 aufgehängt ist. Die Vorrichtung befördert die Dialyselösung aus einem Behälter 89 durch eine Rohrleitung mit Hilfe einer Schlauchpumpe 90 durch ein, an dem Traggestelle 86 befestigte Heizmittel 59 in den Fallbehälter 95. Die Schlauchpumpe 90 ist eine an und für sich bekannte Vorrichtung, welche die Flüssigkeit unter sterilen Umständen weiterleiten kann. Während des Füllungsvorgangs ist ein Ventil 91 geschlosser und die Steuerungseinheit 87 regelt das Heizmittel 59 in der Weise, dass die in den Fallbehälter 95 einfliessende Flüssigkeit im wesentlichen auf einer, für die Einleitung in die Bauchhöhle des Patienten vorgeschriebenen Temperatur ist. Sobald die Flüssigkeit in dem Fallbehälter 95 die vorgeschriebene Menge erreicht hat, stellt die Steuerungseinheit 87 aufgrund des Signals des Kraftmessers 96 die Schlauchpumpe 90 ab, damit ist die Füllung beendet.

Jetzt folgt die Einleitung der sich im Fallbehälter 95 befindenden Dialyseflüssigkeit in die Bauchhöhle des Patienten durch natürliches Gefälle. Zu diesem Zwecke öffnet die Steuerungseinheit 87 die Ventile 91 und 92 nach der zum Patienten führenden Leitung 84 und hält das Ventil 93 geschlossen. Die nicht arbeitende Schlauchpumpe 90 sichert ebenfalls eine Sperrung. Im Laufe des Einleitung steuert die Steuerungseinheit 87 das Heizmittel 59 in der Weise, dass dieses immer die zur Einleitung vorgeschriebene Temperatur aufweist. Demzufolge verlässt die im Fall behälter 95 infolge des eventuellen Wartens schon ein wenig abgekühlte Flüssigkeit das Heizmittel 59 immer an der vorgeschriebenen Temperatur, unabhängig davon, wie die Strömungsgeschwindigkeit sich gestaltet. Die Strömungsgeschwindigkeit kann von zahlreichen Faktoren abhängig sein, z.B. vom Zustand des im Patienten eingeführten Katheters, vom eigenen Zustand des Patienten, usw. Wenn aus dem Fallbehälter 95 die gewünschte Flüssigkeitsmenge schon ausgeflossen ist, was der Kraftmesser 96 anzeigt, schliesst die Steuerungseinheit 87 die Ventile 91 und 92, damit ist der Einleitungsvorgang beendet.

Wenn die Dialyselösung schon genügende Zeit in der Bauchhöhle des Patienten war, ist ihre Ausführung ebenfalls durch natürliches Gefälle erforderlich. Zu diesem Zwecke öffnet die Steuerungseinheit 87 die Ventile 92 und 93, hält das Ventil 91 geschlossen und betätigt nicht die Schlauchpumpe 94. In dieser Weise fliesst die Dialyselösung in einen, an das Traggestelle 86 durch Zwischenschaltung eines Kraftmessers 99 befestigten unteren Aufnahmebehälter 98. Der Kraftmesser 99 dient zur Messung der Menge der aus dem Patienten ausgeführten Dialyseflüssigkeit. Am Ende des Ausführungsvorgangs schliesst die Steuerungseinheit 87 die Ventile 92 und 93.

Danach soll die im Aufnahmebehälter 98 gesammelte Flüssigkeit in den Kanal durch eine Leitung 85 abgelassen werden. Dazu schaltet die Steuerungseinheit 87 die Schlauchpumpe 94 ein, während sie das Ventil 93 geschlossen hält. Am Ende des Ablassens stellt die Steuerungseinheit 87 die Schlauchpumpe 94 ab.

Wenn im Laufe des Einleitungsvorgangs die Steuerungseinheit 87 eine Regelwidrigkeit registriert, z.B. dass die Temperatur des aus dem Heizmittel 59 austretenden Flüssigkeit zu hoch ist, unterbricht sie den Einleitungsvorgang und führt die Flüssigkeit in den Aufnahmebehälter 98. Zu diesem Zwecke schliesst die Steuerungseinheit 87 das Ventil 92 und öffnet das Ventil 93.

Die oben beschriebenen Vorgänge können nicht nur nacheinander, sondern in einzelnen Fällen auch gleich zeitig ablaufen. Dementsprechend kann gleichzeitig mit der Füllung des Fallbehälters 95 die Ausführung der Dialyseflüssigkeit aus dem Patienten geschehen. Gleichzeitig mit der Einleitung der Dialyseflüssigkeit in den Patienten kann sich das Ablassen der früher schon ausgeführten Dialyseflüssigkeit in den Kanal vollziehen.

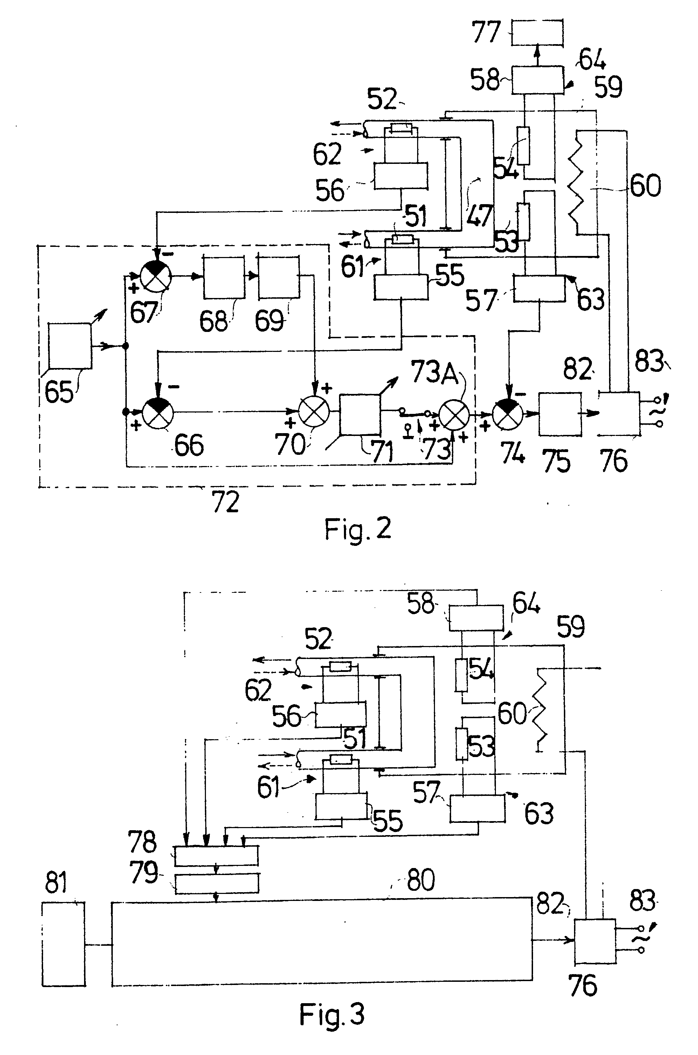

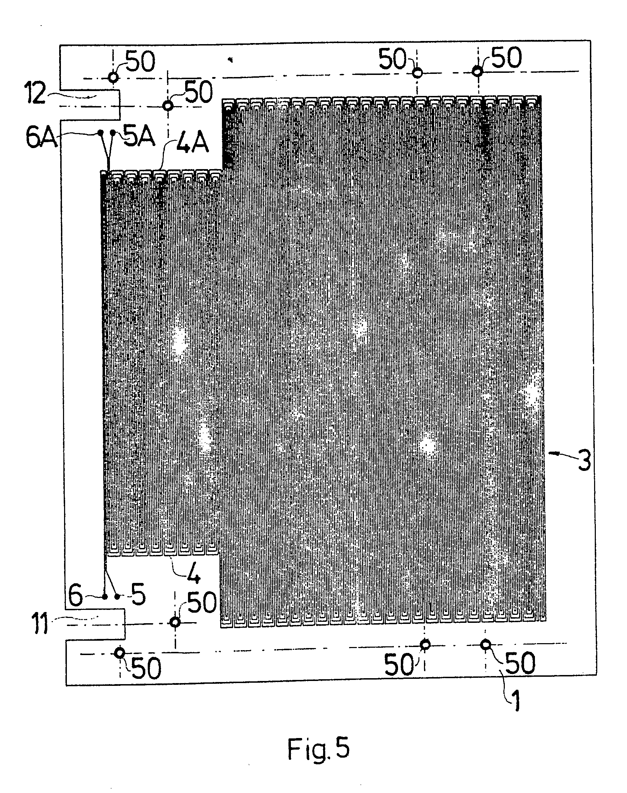

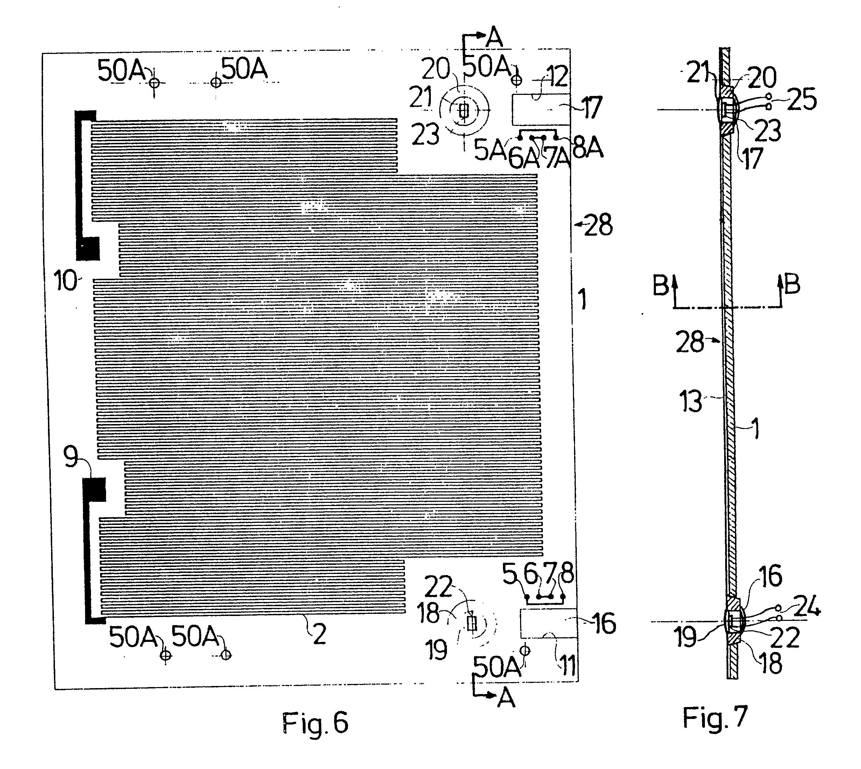

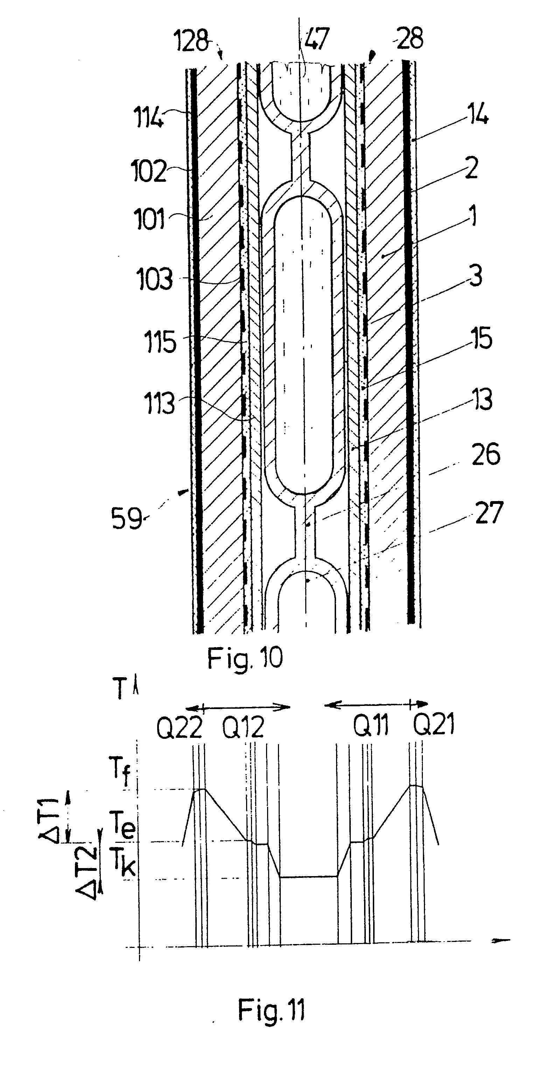

Die erfindungsgemässe Regelung des Heizmittels 59 von Fig. 1 ist in Fig. 2 dargestellt. Das Heizmittel 59 ist mit einem Heizelement 60, einem Kanal 47 für die zu erwärmende Flüssigkeit und zwischen denen angeordneten Temperaturfühlern 53 und 54 versehen. Dem Temperaturfühler 53 schliesst sich ein Signalwandler 57 an, und die beiden bilden einen Signalgeber 63. Dem Temperaturfühler 54 schliesst sich ein Signalwandler 58 an, und die beiden bilden einen Signalgeber 64. Die Temperatur der in den Kanal 47 eintretenden Flüssigkeit ist von einem Temperaturfühler 51 wahrgenommen, dem sich ein Signalwandler 55 anschliesst, und die beiden bilden einen Signalgeber 61. Die Temperatur der aus dem Kanal 47 austretenden Flüssigkeit ist von einem Temperaturfühler 52 wahrgenommen, dem sich ein Signalwandler 56 anschliesst, und die beiden bilden einen Signalgeber 62. Wenn das Heizmittel 59 eine in Fig. 5-10 gezeigte Konstruktion aufweist, ist das Heizelement 6C von den in Reihe geschalteten Heizfoliemustern 2 und 102 (Fig. 6 und 10), der Temperaturfühler 53 von dem in Reihe geschalteten einen Teil der Fühlfoliemuster 3 und 103 (Fig. 5 und 10), und der Temperaturfühler 54 vom dem in Reihe geschalteten anderen Teil der Fühlfoliemuster 3 und 103 gebildet. Dem Temperaturfühler 51 bzw. 52 entspricht der Temperaturfühler 22 bzw. 23 (Fig. 6 und 7). Die Signalwandler 55, 56, 57 und 58 können von Wechselspannung gespeiste Brükkenwiderstandsmesser sein.

Die Temperaturregelung des Heizmittels 59 geschieht durch einen Regelungskreis, welcher ein Differenzglied 74, eine zum dessen Ausgang angelegte Regeleinheit 75, Vorteilhaft einen PI-Regler, und eine dem Ausgang 82 der Regeleinheit 75 angeschlossene Heizelementspeise einheit 76 enthält, dem Ausgang dieser letzteren ist das Heizelement 60 angeschlossen. Die Heizelementspeiseeinheit 76 bekommt 220 V Wechselspannung von Netzklemmen 83, und regelt die Heizung des Heizelements 60 in der Weise, dass sie das Heizelement 60 mal unter Netzspannung setzt, mal vom Netz abschaltet. Die Regeleinheit 75 soll also am Ausgang 82 Steuersignal vom ja-nein Charakter abgeben. Solche Heizelementspeiseeinheit 76 kann mit einem an und für sich bekannten Halbleiter-Thyristorrelais ausgeführt werden, welches die Ein- und Ausschaltung immer bei dem nächsten Nullübergang durchführt. Zum positiven Eingang des Differenzglieds 74 gibt eine Sollwerteinheit 72 den Temperatursollwert. Dem negativen Eingang des Differenzglieds 74 ist der Ausgang des Signalgebers 63 angeschlossen.

Die Sollwerteinheit 72 enthält einen Sollwertgeber 65, mit welchem die, der vorgeschriebenen Temperatur der Flüssigkeit entsprechende Temperatur des Heizmittels eingestellt werden kann. Der Ausgang des Sollwertgebers 65 ist einerseits einem positiven Eingang der Differenzglieder 66 und 67, andererseits einem Eingang eines Addierungsglieds 73A angeschlossen. Der negative Eingang des Differenzglieds 66 ist dem Ausgang des Signalgebers 61 angeschlossen und der negative Eingang des Differenzglieds 67 ist dem Ausgang des Signalgebers 62 angeschlossen. Der Ausgang des Differenzglieds 66 schliesst sich zum einen Eingang eines Addierungsglieds 70, während der Ausgang des Differenzglieds 67, durch Zwischenschaltung einer Regeleinheit 68, vorteilhaft eines PID-Reglers, und eines Begrenzers 69 zum anderen Eingang des Addierungsglieds 70 an. Der Ausgang des Addierungsglieds 70 schliesst sich durch einen Verstärker 71 mit einstellbarer Verstärkung und eine Klemme des Umschalters 73 zum anderen Eingang des Addierungsglieds 73A an. Die andere Klemme des Umschalters 73 ist zum Erdpunkt angeschlossen. Der Ausgang des Differenzglieds 73A gibt das Sollwertsignal zum positiven Eingang des Differenzglieds 74.

Das Regelungssystem arbeitet wie folgt. In der in Fig. 2 gezeichneten Stellung des Umschalters 73 geschieht die Füllung des Fallbehälters 95 bei der Vorrichtung nach Fig. 1. Da fliesst die Flüssigkeit, dem mit kontinuierlicher Linie bezeichneten Pfeil entsprechend, in das Heizmittel 59 ein bzw. aus, d.h. der Signalgeber 61 misst die Temperatur der einfliessenden Flüssigkeit, während der Signalgeber 62 die Temperatur der ausfliessenden und so in den Fallbehälter 95 ankommenden Flüssigkeit. Es ist ersichtlich, dass in diesem Falle das Differenzglied 66 an seinem Ausgang ein Signal auf den Eingang des Verstärkers 71 gibt, welches der Differenz zwischen der vorgeschriebenen Temperatur der ausfliessenden Flüssigkeit und der tatsächlichen Temperatur der einfliessenden Flüssigkeit entspricht. Die Verstärkung des Verstärkers 71 ist dem Massenstrom und der spezifischen Wärme der durch das Heizmittel 59 durchfliessenden Flüssigkeit, sowie den Parametern des Heizmittels 59 entsprechend eingestellt. An seinem Ausgang gibt der Verstärker 71 ein solches Sollwertsignal dem Regelungskreis des Temperaturreglers des Heizmittels 59, welches in dem Heizmittel 59 eine, die vorgeschriebene Temperatur der austretenden Flüssigkeit sichernde Heizung ergibt. Im Falle einer gegebenen spezifischen Wärme und eines gegebenen Massenstroms und für ein gegebenes Heizmittel 59 kann die Verstärkung des Verstärkers 71 durch Messung oder Berechnung bestimmt werden. Diese steuertechnische Einstellung der Heizung des Heizmittels 59 wird aber in dem tatsächlichen Falle die Temperatur der ausfliessenden Flüssigkeit nicht genau auf die vorgeschriebene Temperatur einstellen, es ist deshalb vorteilhaft, wenn das Differenzglied 67 das Ausgangssignal des Signalgebers 62 mit einem Signal proportional zur vorgeschriebenen Temperatur vergleicht, und an seinem Ausgang durch die Regeleinheit 68 und den Begrenzer 69 ein dieser Differenz entsprechendes Signal auf einen Eingang des Addierungsglieds 70 gibt. Dadurch wird das am Ausgang des Differenzglieds 66 erscheinende Signal und auch das eingestellte Sollwertsignal dementsprechend fortlaufend geändert, wie die Temperatur der austretenden Flüssigkeit von der vorgeschriebenen Temperatur abweicht. Der Begrenzer 69 begrenzt das Ausgangssignal der Regeleinheit 68 in positiver und negativer Richtung gleicherweise, um es zu erreichen, dass das vom Differenzglied 66 eingestellte Sollwertsignal nur bis zu einem bestimmten Masse geändert werden könne. Dieses Mass ist kleiner als ± 30 %, vorteilhaft kleiner als ± 10 %. Mit der Begrenzung kann es gesichert werden, dass bei transienten Vorgängen, z.B. bei dem Beginn der Temperaturregelung der Flüssigkeit, das Regelungssystem weniger einschwingt. Dieses Regelungssystem sichert, dass der Fallbehälter 95 mit grosser Geschwindigkeit gefüllt werden kann, wodurch die Behandlungsdauer bzw. die Wartezeit des Patienten verkürzt werden kann, und gleichzeitig die Temperatur der in den Fallbehälter 95 eintretenden Flüssigkeit der vorgeschriebenen Temperatur der Einleitung in den Patienten im wesentlichen entspricht.

Wenn im Fallbehälter 95 die Flüssigkeitsmenge das vorgeschriebene Mass erreicht, was vom Kraftmesser 96 angezeigt ist, schaltet die Steuerungseinheit 87 erst die Heizung des Heizelementes 60 aus, der Betrieb der Schlauchpumpe 90 wird aber vorläufig fortgesetzt. Wenn im Heizmittel 59 die vom Signalgeber 63 wahrgenommene Temperatur auf die für die Einleitung in den Patienten vorgeschriebene Temperatur abnimmt, stellt die Steuerungseinheit 87 die Schlauchpumpe 90 ab und das Regelungssystem schaltet auf die werterhaltende Regelung um. Das ist in Fig. 2 durch die Umschaltung des Umschalters 73 symbolisiert. Das Sollwertsignal wird nun unmittelbar vom Sollwertgeber 65 gegeben. Danach - gegebenenfalls mit der Zwischenschaltung einer Wartepause -beginnt die Einleitung der Flüssigkeit in den Patienten aus dem Fallbehälter 95. In Fig. 2 fliesst jetzt die Flüssigkeit, dem mit Strichellinie gezeigten Pfeil entsprechend, durch das Heizmittel 59, in welchem die der im Kanal 47 strömenden Flüssigkeit nahe liegenden Teile auf die vorgeschriebene Temperatur der Flüssigkeit geregelt sind. Es ist dadurch gesichert, dass der Temperaturfühler 53 an diesen Teilen angeordnet ist, wie es bei den Fühlfoliemustern 3 und 103 zu sehen ist (Fig. 10).

Die Fühlfoliemuster 3 und 103 ergeben zwei voneinander unabhängige Temperaturfühler. Der eine ist der Temperaturfühler 53, der andere der Temperaturfühler 54. Der Ausgang des Signalgebers 64 des letzteren ist einer Ausschalt- und Alarmeinheit 77 angeschlossen, deren Aufgabe ist, die Vorrichtung auszuschalten und Alarmsignal abzugeben, wenn die Temperatur im Heizmittel 59 sich aus jedwedem Grunde, z.B. im Falle einer Betriebsstörung, über einen vorbestimmten Wert erhöht. Diese Ausschaltung ist bei der Vorrichtung nach Fig. 1 von der Steuerungseinheit 87 durchgeführt. Wenn z.B. dieser Gefahzustand während der Einleitung in den Patienten vorkommt, wird die Einleitung durch Schliessen des Ventils 92 unterbrochen, und durch Öffnung des Ventils 93 fliesst die Flüssigkeit in der unteren Aufnahmebehälter 98.

Eine vorteilhafte Ausführungsform des Temperaturregelungssystems des Heizmittels 59 ist in Fig. 3 dargestellt. Bei dieser Lösung geschieht die Temperaturregelung in digitaler Weise. Die Ausgänge der Signalgeber 61, 62, 63 und 64 kommen durch einen Messpunkwandler 78 zum Eingang eines analog-digitalen Umwandlers 79, dessen Ausgang einer digitalen Datenverarbeitungsvorrichtung 80 angeschlossen ist. Die Datenverarbeitungsvorrichtung 80 ist mit einen Bedienungs- und Anzeigeeinheit 81 versehen und weist einen Ausgang 82 auf, welcher die Heizelementspeiseeinheit 76 steuert. Die Datenverarbeitungsvorrichtung 80 ist vorteilhaft mit einem Mikroprozessor versehen und ist derart programmiert, dass sie die Regelung gemäss Fig. 2 ver wirklicht. Die Datenverarbeitungsvorrichtung 80 kann selbstverständlich noch überdies weitere sonstige Aufgaben erfüllen, so z.B. verrichtet sie auch die Aufgabe der in Fig. 2 gezeigten Ausschalt- und Alarmeinheit 77. Das Heizungsregelungssystem kann aber auch in analoger Weise, durch analoge Realisierung der in Fig. 2 dargestellten Elementen verwirklicht werden.

In Fig. 4 ist ein anderes vorteilhaftes Regelungssystem des Heizmittels der Vorrichtung gemäss Fig. 1 dargestellt. Die Bezugszeichen entsprechen den Bezugszeichen der Fig. 2, sodass hier nur die Unterschiede erwähnt werden. Das Regelungssystem der Fig. 4 weicht insofern von dem Regelungssystem der Fig. 2 ab, dass hier dem Sollwertsignal nicht eine bestimmte Temperatur im Heizmittel 59, sondern ein vom Heizelement 60 nach der im Kanal 47 strömenden Flüssigkeit richteter bestimmter Wärmefluss entspricht. Zu diesem Zwecke sind zwischen dem Heizelement 60 und dem Kanal 47 an zwei, in der Richtung des Wärmeflusses nacheinander liegenden Stellen zwei Temperaturfühler 53A und 53B angeordnet. Der Temperaturfühler 53A bildet mit einem Signalwandler 57A einen Signalgeber 63A, während der Temperaturfühler 53B mit einem Signalwandler 57B einen Signalgeber 63B. Der Ausgang des Signalgebers 63A ist einem positiven Eingang eines Differenzglieds 48, der Ausgang des Signalgebers 63B dagegen durch einen Umschalter 49 einem negativen Eingang des Differenzglieds 48 angeschlossen. Der Ausgang des Differenzglieds 48 schliesst sich zum negativen Eingang des Differenzglieds 74 an. Der Ausgang des Differenzglieds 74 ist durch die Regeleinheit 75 dem Eingang der Heizelementspeiseeinheit 76 angeschlossen, welche das Heizelement 60 z.B. mit Gleichstrom speist. Wenn das Heizmittel 59 nach Fig. 5-10 ausgeführt ist, entsprechen die in Reihe geschalteten Heizfoliemuster 2 und 102 (Fig. 6 und 10) dem Heizelement 60, während der in Reihe geschaltete eine Teil der Fühlfoliemuster 3 und 103 (Fig. 5 und 10) dem Temperaturfühler 53A. Selbst die in Reihe geschalteten Heizfoliemuster 2 und 102 können den Temperaturfühler 53B gestalten. Von der Gleichstromheizung kann die Brückenwiderstandsmessung mit Wechselspannung von einigen kHz leicht getrennt werden. Der Temperaturfühler 53B kann aber auch in der Weise ausgeführt werden, dass neben dem Heizfoliemuster 2 bzw. 102, an der gleichen Seite der Tragplatte 1 bzw. 101 eine weitere Fühlmäanderformation ausgestaltet ist, ähnlicherweise wie bei den gegenseitig hineinragenden Mäanderformationen 4 und 4A (Fig. 5).

Ein weiterer Unterschied im Verhältnis zur Fig. 2 ist die Messung des Massenstroms der bei der Füllung des Fallbehälters 95 einfliessenden Flüssigkeit durch einen Massenstromfühler 51A und einen Signalwandler 55A, dessen Ausgang einem Steuereingang des Verstärkers 71 angeschlossen ist. Die Verstärkung des Verstärkers 71 ist damit so beeinflusst, dass einem grösseren Massenstrom eine grössere Verstärkung gehört. Das Regelungssystem nach Fig. 4 kann aber ohne diese Massenstrommessung auch funktionieren.

Das Regelungssystem nach Fig. 4 regelt die Temperatur der Flüssigkeit in der angeführten Stellung der Umschalter 49 und 73 während der Fülung des Fallbehälters 95. Die Flüssigkeit fliesst nun in der Richtung der mit kontinuierlichen Linie bezeichneten Pfeile durch den Kanal 47. Das Ausgangssignal des Differenzglieds 48 ist proportional zur Differenz zwischen den von den Temperaturfühlern 53B und 53A gemessenen Temperaturen und so zum vom Heizelement 60 nach der Flüssigkeit fliessenden Wärmefluss. In der Sollwerteinheit 72 muss also das Sollwertsignal durch den Verstärkers 71 so eingestellt werden, dass der von ihm bestimmte Wärmefluss - bei gegebenem Massenstrom - die Temperatur der eintretenden Flüssigkeit bis zum Austritt auf die vorgeschriebene Temperatur erhöht. Während der Einleitung aus dem Fallbehälter 95 in den Patienten, wenn die Flüssigkeit den gestrichelten Pfeilen entsprechend fliesst, sind die Umschalter 49 und 73 in der anderen Stellung. Nun funktioniert dieses Regelungssystem ebenso, wie dasjenige gemäss Fig. 2, da der Temperaturfühler 53B ausgeschaltet ist und der Temperaturfühler 53A dem Temperaturfühler 53 entspricht.

Obwohl in Fig. 1-4 eine Peritonealdialysevorrichtung beschrieben wurde, kann die Erfindung auch zur Einführung jedweder physiologischen Flüssigkeit, z.B. Bluts oder Infusionslösung, verwendet werden.

In Fig. 5-7 ist eine Seitenwand 28 des in der erfindungsgemässen Vorrichtung gut anwendbare Heizmittels und eine Tragplatte 1 der Seitenwand 28 dargestellt. An einer Seite der aus Isoliermaterial hergestellten Tragplatte 1 (Fig. 6) ist ein, eine Mäanderformation bildendes Heizfoliemuster 2 ausgestaltet, zu dessen Ende Stromzufuhrflächen 9 und 10 angeschlossen sind. An der anderen Seite der Tragplatte 1 (Fig. 5) ist ein, zwei Mäanderformationen 4 und 4A bildendes Fühlfoliemuster 3 ausgestaltet, welches als zwei temperaturfühlende Widerstände dient. Die Mäanderformation 4 hat Endungen 5 und 6, die Mäanderformation 4A Endungen 5A und 6A. In der Fig. 5 ist es ersichtlich, dass die zwei Mäanderformationen 4 und 4A kammartig ineinanderreichen und beinahe gleichmässig die Oberfläche der Tragplatte 1 decken. Das Heizfoliemuster 2 an der anderen Seite der Tragplatte 1 bildet ebenfalls eine solche Mäanderformation, welche beinahe gleichmässig die Oberfläche der Tragplatte 1 bedeckt, sodass ein praktisch gleichmässige Heizung der Tragplatte 1 gesichert ist. Die Mäanderformationen des Heizfoliemusters 2 und des Fühlfoliemusters 3 sind aufeinander senkrecht angeordnet, um einerseits eine induktive Kopplung an je kleinerem Wert zu halten, andererseits eine bessere Temperaturfühlung zu sichern. Diese Anordnung ermöglicht eine sehr genaue Ermittlung der Durchschnittstemperatur der Oberfläche der Tragplatte 1 unabhängig von der relativen Position des Heizfoliemusters 2 und des Fühlfoliemusters 3. Das Heizfoliemuster 2 kann vorteilhaft von Netzspannung gespeist werden, und die Mäanderformationen 4 und 4A des Fühlfoliemusters 3 können zu je einem, durch eine Spannung von höherer Frequenz als die Netzspannung, z.B. von einigen kHz, gespeisten Brückenstromkreis angeschlossen werden.

Das Heizfoliemuster 2 ist sehr dünn, folgenderweise ist seine wärmeabgebende Oberfläche im Verhältnis zu seinem Querschnitt gross, demzufolge erträgt es eine grosse Oberflächenleistungsdichte. Der Querschnitt soll so gewählt werden, dass bei der zur Verfügung stehenden Speisespannung die nötige Heizleistung gesichert sei. In einer vorteilhaften Ausführungsform ist die Mäanderformation aus 35 µm dicker Kupferfolie hergestellt, wo die Breite der geraden Foliestrecken 0,7 mm ist, der Widerstand des ganzen Heizfoliemuster 24 Ohm ausmacht, die Heizspannung 110 V und die Heizleistung 400 W ist. Wenn in einem Heizmittel zwei solche Heizfoliemuster 2 miteinander in Reihe geschaltet sind, kann das Heizmittel von einer 220 V Netzspannung gespeist werden. Zwecks Regelung soll die Heizleistung selbstverständlich regelbar sein.

Das Fühlfoliemuster 3 kann ebenfalls aus 35 µm dicker Kupferfolie hergestellt werden, wo die geraden Foliestrecken 0,35 mm breit sind, während der Widerstand 2 x 50 Ohm ist. Diese Ausführung ergibt einen doppelten Temperaturfühler, welcher infolge der Sicherheitsvorschriften für medizinische Geräte erforderlich sein kann. Gegebenenfalls kann auch ein, einen einziger Temperaturfühler bildendes Fühlfoliemuster 3 ausreichend sein.

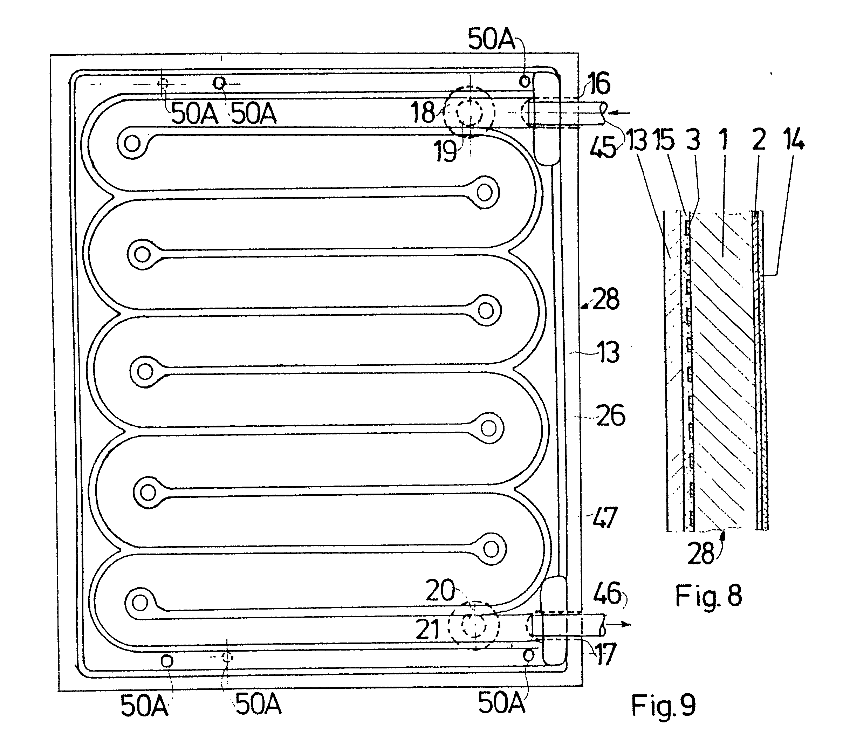

Das Heizfoliemuster 2 kann wunschgemäss auch als Fühlfoliemuster verwendet werden, z.B. in der Weise, dass die Heizung mit Gleichspannung gesichert ist, während die Temperaturfühlung (Widerstandsmessung) in einer mit Wechselspannung gespeisten Messbrücke geschieht. Es ist auch möglich, dass neben dem Heizfoliemuster 2 ein weiteres Fühlfoliemuster angeordnet ist, z.B. derart, dass die zwei Mäanderformationen elektrisch voneinander getrennt sind, aber ähnlich wie das Fühlfoliemuster kammartig ineinanderreichen. Diese Lösung ist von Bedeutung, wenn man die Temperatur an beiden Seiten der isolierenden Tragplatte 1, z.B. zwecks Wärmeflussmessung und -regelung nach Fig. 4, messen will. Aus der Anordnung ist es ersichtlich, dass das Fühlfoliemuster 3 im wesentlichen die Durchschnittstemperatur der ganzen Oberfläche der Tragplatte 1 misst. Die Endungen 5, 6 und 5A, 6A sind durch metallisierte Bohrungen zur anderen Seite der Tragplatte 1 durchgeführt und dort mit Folienstrecken zu Lötstellen 7, 8 bzw. 7A, 8A angeschlossen. In dieser Weise können die elektrischen Anschlüsse sowohl zum Heizfoliemuster 2, als auch zum Fühlfoliemuster 3 von der, in Fig. 6 dargestellten Seite geschehen. An der mit Fühlfoliemuster 3 versehenen Seite der Tragplatte 1 (Fig. 5) sind aus Folie bestehende Ringchen 50 ausgeformt, welche die Positionen der in Fig. 6 sichtbaren Positionsbohrungen 50A und Temperaturfühler 22 und 23 bezeichnen. Die Tragplatte 1 ist mit zwei Einschnitten 11 und 12 versehen, welche zur Aufnahme der Einfluss- und Ausflussöffnungen des später in Zusammenhang mit Fig. 9 zu beschreibenden Beutels 26 dienen.

Die Tragplatte 1 kann vorteilhaft aus zweiseitig folierter, mit Glasfaser verstärker gedruckter Schaltungsplatte mit einer Dicke von etwa 1,5 mm ausgestaltet werden, und die Foliemuster können mit der gewöhnlichen Ätztechnologie gedruckten Schaltungen hergestellt werden. Diese gewöhnlichen gedruckten Schaltungsplatten sind verwendbar, wenn die Temperatur des Heizfoliemusters 2 nicht höher als 100 °C ist. Um eine höhere Temperatur zu ertragen, ist eine andere Tragplatte 1 mit einem Heizfoliemuster 2 erforderlich. Eine auf Keramikplättchen aufgetragene dünnschichtige Leitfolie kann z.B. gut verwendet werden. Die auf die zwei Seiten der Tragplatte 1 aufgetragenen Foliemuster können auch aus verschiedenen Materialien hergestellt werden. Es ist vorteilhaft, wenn der Widerstand des Heizfoliemuster 2 nur wenig von der Temperatur abhängig ist. Das Fühlfoliemuster 3 dagegen soll zweckmässigerweise aus einem Material von je grösserer Temperaturabhängigkeit hergestellt werden. Die Foliemuster können auch durch Vakuumverdampfung auf die Tragplatte 1 aufgebracht werden.

In Fig. 6-8 ist die Seitenwand 28 im zusammenbauten Zustand dargestellt. In Fig. 8 ist es ersichtlich, dass auf eine Seite der Tragplatte 1 das Heizfoliemuster 2 aufgetragen ist, dieses mit einer Schutzschicht 14 bedeckt ist, welche das Heizfoliemuster 2 von Oxydation und äusseren Einwirkungen schützt und gleichzeitig als eine Isolierschicht wirkt. Die Schutzschicht 14 kann z.B. der in der Technologie der gedruckten Schaltungen übliche durchsichtige Solder-Lack sein mit einer Dicke von etwa 0,05 mm. Auf die andere Seite der Tragplatte 1 ist das Fühlfoliemuster 3 aufgetragen. Darauf ist eine Temperaturausgleichschicht 13 durch eine verklebende Isolierschicht 15 befestigt. Die Temperaturausgleichschicht 13 soll aus einem Material mit guter Wärmeleitfähigkeit, z.B. aus Aluminium oder aus Kupfer hergestellt werden. Die Dicke der Temperaturausgleichschicht 13 soll so gewählt werden, dass sie die Temperaturunterschiede, die infolge der Ausgestaltung und Position des Fühlfoliemusters 2 und des Inhomogenitäts in der Wärmleitfähigkeit des zu erwärmenden Mediums auftreten, ausgleicht ohne die Wärmeträgheit des Heizmittels übermässig zu vergrössen. In der gezeigten Ausführungsform ist eine Aluminiumplatte mit einer Dicke von C,5 mm geeignet. Die Isolierschicht 15 kann z.B. aus mit Glasgewebe verstärktem Polyesterharz mit einer Dicke von etwa 0,1 mm bestehen, welche bei etwa 170 °C auf die, mit dem Fühlfoliemuster 3 bedeckte Seite der Tragplatte 1 aufgepresst werden kann. Dieses Befestigungsverfahren kann bis einer Betriebstemperatur von etwa 120 °C verwendet werden.

Fig. 9 zeigt eine Ansicht der Seitenwand 28 von der Temperaturausgleichschicht 13 her gesehen, mit einem auf die Temperaturausgleichschicht 13 gelegten Beutel 26. Die vom Heizmittel zu erwärmende Flüssigkeit strömt in einem, im Beutel 26 ausgeformten mäanderförmigen Kanal 47. Der Kanal 47 schliesst sich zu einer Einflussöffnung 45, sowie zu einer Ausflussöffnung 46 an. Der Beutel 26 kann durch Verschweissung von zwei Kunststoffolien hergestellt werden, und die zwischen den zwei Kunststoffolien eingeschweissten Rohre bilden die Einflussöffnung 45 und die Ausflussöffnung 46. Zur Einspannung dieser Rohre sind in der Temperaturausgleichschicht 13 zylindrische Prägungen 16 und 17 vorgesehen. Die Positionierung des Beutels 26 ist durch Löcher gesichert, die an dem Beutel 26 mit den Ringchen 50 entsprechenden Stellen ausgeformten Bohrungen 50A übereinstimmend ausgestaltet sind. Für die Messung der Temperatur der in den Beutel 26 eintretenden Flüssigkeit ist in einem, in der Tragplatte 1 befestigten Teflonring 18 eine kugelhaubenförmige Metallplatte 19 hineinspringen gelassen, an deren dem Beutel 26 gegenüberliegenden Seite ein Temperaturfühler 22 befestigt ist. Der Temperaturfühler 22 kann z.B. ein Platinwärmewiderstand sein, welcher mit Ausführungen 24 versehen ist. Es ist in Fig. 6 zu sehen, dass der Teflonring 18 in entsprechenden Entfernung von dem Heizfoliemuster 2 angeordnet ist. Er hat die Aufgabe, zwischen der Metallplatte 19 einerseits und der Tragplatte 1 sowie der Temperaturausgleichschicht 13 anderseits eine gute Wärmeisolierung zu sichern. Für die Messung der Temperatur der aus dem Beutel 26 austretenden Flüssigkeit ist in einem ähnlichen Teflonring 20 eine mit Temperaturfühler 23 versehene Metallplatte 21 angeordnet. Der Temperaturfühler 23 hat Ausführungen 25. Die Anordnung ist vollkommen symmetrisch, so ist es durch die Strömungsrichtung der Flüssigkeit bestimmt, welcher von den Temperaturfühlern 22 und 23 die Temperatur der eintretenden und welcher die der austretenden Flüssigkeit messen wird. Die Metallplatten 19 und 21 können aus z.B. anodisch oxydiertem Aluminium mit einem Durchmesser von 13 mm und einer Dicke von 0,3 mm her gestellt werden. Auf ihre Rückseite kann z.B. je ein auf 1 mm² grosses Keramikplättchen aufgetragener Platinwärmewiderstand aufgeklebt werden.

In Fig. 10 ist ein Teil der zwei Seitenwände 28 und 128 des Heizmittels 59, sowie der des zwischen den Seitenwänden 28 und 128 angeordneten Beutels 26 sichtbar. Im Beutel 26 fliesst die zu erwärmende Flüssigkeit 27 in einem mäanderförmigen Kanal 47. Die Bezugzeichen der Elemente der Seitenwand 128 entsprechen den bei der Seitenwand 28 angewandten Bezugzeichen mit dem Unterschied, dass sie um 100 grösser sind. In der Seitenwand 128 sind die, die Temperatur der in den Beutel 26 eintretenden und daraus austretenden Flüssigkeit fühlenden Signalgeber nicht eingebaut, sonst entspricht die Konstruktion dem Aufbau der Seitenwand 28.

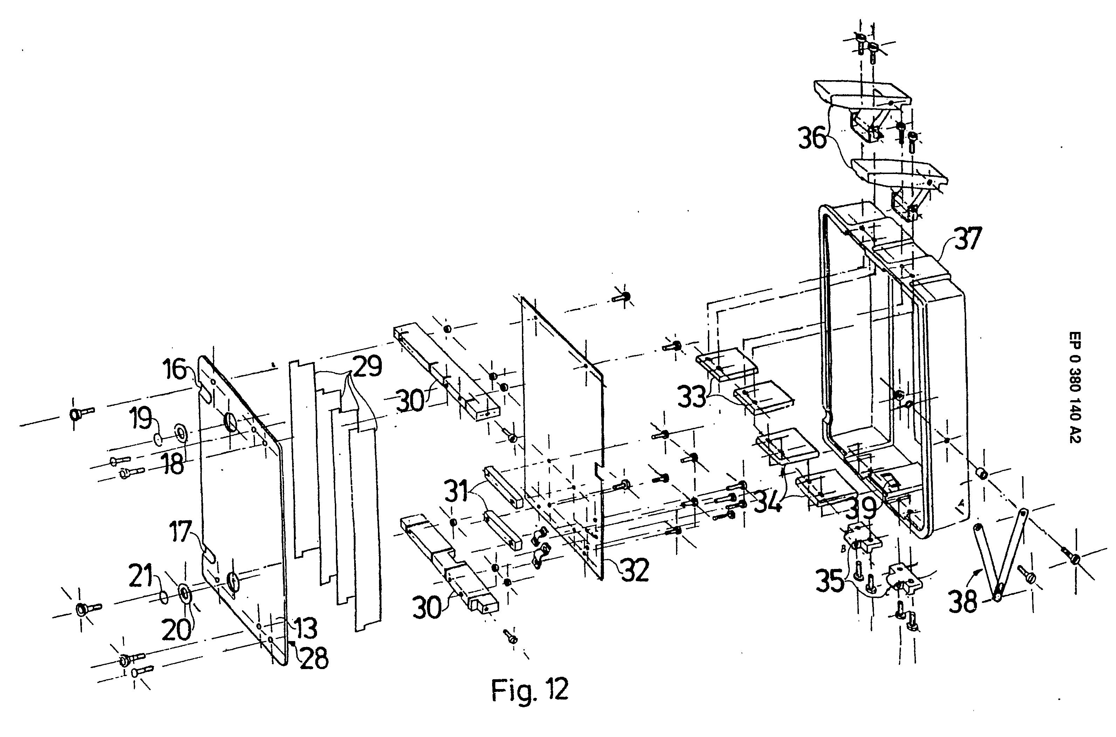

Fig. 11 veranschaulicht die im Heizmittel 59 nach Fig. 10 erscheinenden Temperaturverhältnisse. Das Diagramm zeigt die in den einzelnen Schichten des Heizmittels 59 erhaltenen Durchschnittswerte der Temperatur T. Es ist sichtbar, dass die höchste Temperatur $T_f$ bei den Heizfoliemustern 2 und 102 auftritt. Von hier fliesst einerseits der Wärmefluss Q11 bzw. Q12 der Flüssigkeit 27 zu, andererseits der einen Verlust verursachende Wärmefluss Q21 bzw. Q22 auswärts an. Wegen der Temperatursenkung in den Tragplatten 1 und 101 fühlen die Fühlfoliemuster 3 und 103 durchschnittlich eine Temperatur $T_e$. Zwischen den Temperaturen $T_f$ und $T_e$ ergibt sich eine Temperaturdif-

ferenz ΔT1. Es entsteht eine kleine Wärmestufe in den Isolierschichten 15 und 115, während in den Temperaturausgleichschichten 13 und 113 sich praktisch keine Wärmestufe ergibt. Es ist offensichtlich, dass die Temperatur der Temperaturausgleichschichte 13 und 113 praktisch mit der Temperatur $T_e$ übereinstimmt. An der Kunststoffwand des Beutels 26 entsteht eine weitere Temperatursenkung und dementsprechend bildet sich die durchschnittliche Temperatur $T_k$ der Flüssigkeit 27. Zwischen den Temperaturen $T_e$ und $T_k$ ergibt sich eine Temperaturdifferenz ΔT2.

Im Heizmittel 59 gemäss Fig. 10 fühlen die Fühlfoliemuster 3 und 103 die auf die ganze Seitenwand 28 bzw. 128 bezogene Durchschnittstemperatur. Die tatsächliche Temperatur längs des Fühlfoliemusters 3 und 103 ergibt sich der Tatsache entsprechend, welchen Wärmeentzug die im Kanal 47 strömende Flüssigkeit bei den einzelnen Punkten der Tragplatte 1 bzw. 101 bedeutet. Demgemäss nimmt die Temperatur von der Eintrittsstelle nach der Austrittsstelle der Flüssigkeit immer zu. In der strömenden Flüssigkeit können aber Strömungsungleichmässigkeiten und auch Blasen entstehen, und die letzteren, in dem mäanderförmigen Kanal 47 steckenbleibend, verursachen lokale Erwärmungen. Zwecks Verhinderung dieser Erscheinung sind die Temperaturausgleichschichte 13 und 113 vorgesehen, dadurch sind sowohl den Beutel 26, als auch die Seitenwände 28 und 128 geschützt.

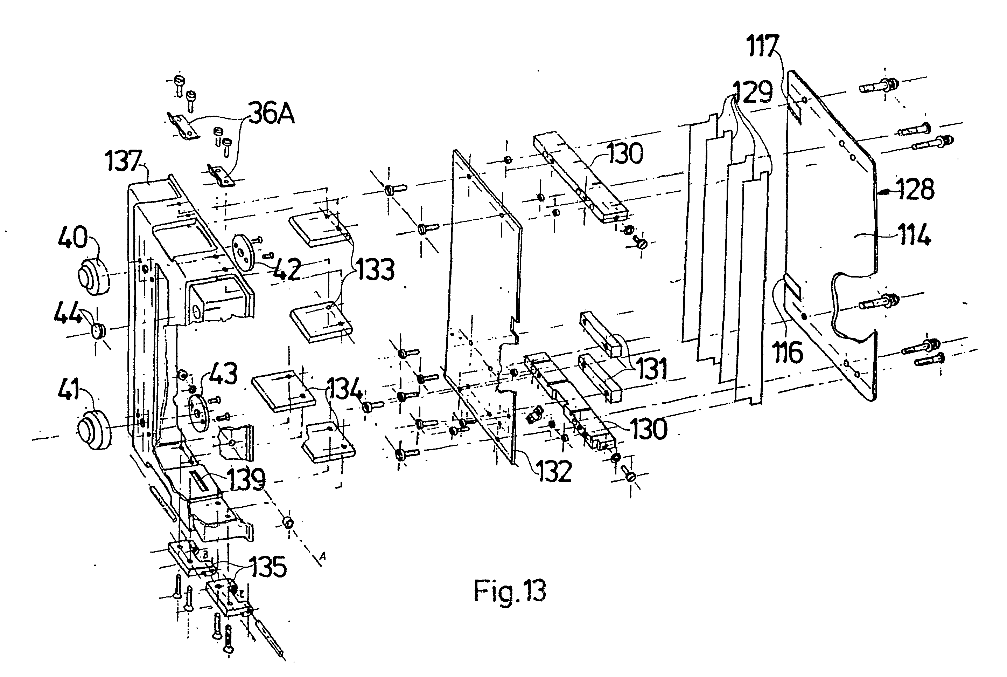

In Fig. 12 bzw. 13 ist je eine, die Seitenwand 28 bzw. 128 des Heizmittels 59 enthaltende Armatur dargestellt. Die Seitenwand 28 (Fig. 12), die aus Isolierstoff hergestellten Verstarkungsrippen 29, die Feststellelemente 30, sowie eine Montageplatte 32 von gedruckter Schaltung sind zueinander mit Schrauben befestigt. An einer der Verstärkungsrippen 29, welche mit gedruckter Schaltung versehen ist, sind die Widerstände der, zum Temperaturfühlfoliemuster 3, sowie zu den an den Metallplatten 19 und 21 angeordneten Temperaturfühlern 22 und 23 gehörigen Brückensignalwandler angeordnet. Die elektrische Verbindung ist von einem, bei der Montage sich schliessenden lösbaren Anschluss gesichert, deren eine Hälfte an der mit gedruckter Schaltung versehenen Verstärkungsrippe 29, die andere Hälfte an der mit gedruckter Schaltung versehenen Montageplatte 32 befestigt ist. Diese Einrichtung ist in zusammengebautem Zustand in einem Gehäuse 37 aus Kunststoff angeordnet und z.B. bei der Kante mit Verklebung in der Weise befestigt, dass die Kante des Gehäuses 37 mit der äusserer Fläche der Temperaturausgleichschicht 13 der Seitenwand 28 in gleicher Ebene liegt. An dem Gehäuse 37 ist unten eine Öffnung 39 vorgesehen, wodurch das die elektrische Verbindung sichernde Bandkabel eingeführt ist, dessen Ende zur, mit gedruckter Schaltung versehenen Montageplatte 32 gelötet und dazu mit Andrückelementen 31 angesetzt ist. Zu dem Gehäuse 37 sind unter Gelenkbänder 35 mit Feststellplättchen 34 und Schrauben befestigt, während oben zwei Exzenterverschlüsse 36 mit Feststellplättchen 33 und Schrauben angeschlossen sind.

Die Gelenkbänder 35 des Gehäuses 37 schliessen sich zu den, mit Hilfe von Feststellplättchen 134 befestigten Gelenkbänder 135 des Gehäuses 137 (Fig. 13) an und ermöglichen, dass die zwei Gehäusen 37 und 137 im Verhältnis zueinander umkippbar sind, höchstens bis zu einem, von Abstandshalter 38 erlaubten Masse von z.B. 90°. Im Gehäuse 137 ist die aus der Seitenwand 128, Verstärkungsrippen 129, Feststellelementen 130 und der mit gedruckter Schaltung versehenen Montageplatte 132 bestehende Montageeinheit befestigt, welche ebenfalls mit Schrauben verbunden ist. Am Oberteil des Gehäuses 137 sind Zungen 36A mit Hilfe von Feststellplättchen 133 befestigt. Zu diesen Zungen 36A schliessen sich die Exzenterverschlüsse 36 in dem verschlossenen Zustand der zwei Gehäusen 37 und 137 an, in welchem Zustand zwischen ihnen eine Spalte von einigen mm zwecks Anordnung des, von der zu erwärmenden Flüssigkeit durchgeflossenen Beutels 26 vorgesehen ist. Die elektrische Verbindung der Seitenwand 128 ist in gleicher Weise wie die der Seitenwand 28 ausgestaltet, da kommt das Bandkabel durch die am Unterteil des Gehäuses 137 ausgeformte Öffnung 139 ein, und das Bandkabel ist zur Montageplatte 132 mit Andrückelementen 141 angeschlossen. Die Befestigungsknöpfe 40 und 41 sind an dem Gehäuse 137 mit scheibenförmigen Feststellplättchen 42 und 43 sowie mit Schrauben befestigt. Die Befestigungsknöpfe 40 und 41 dienen zur Befestigung des kompletten Heizmittels 59. An dem Gehäuse 137 befindet sich noch ein Heizungsanzeiger 44, welcher in dem Falle leuchtet, wenn die Heizfoliemuster 2 und 102 der Seitenwände 28 und 128 eingeschaltet sind.

Die in Fig. 10, 12 und 13 dargestellte Ausführungsform des Heizmittels 59 ist bei medizinischer Anwendung besonders vorteilhaft, wo die Sterilität der physiologischen Flüssigkeit auch nach dem Durchfluss durch das Heizmittel 59 gesichert werden muss. Die Erfindung ist aber nicht auf diese Ausführungsform beschränkt. Es ist leicht einzusehen, dass die Begrenzungswände des zwischen den Seitenwänden 28 und 128 angeordneten Kanals 47 selbst die Temperaturausgleichschichte 13 und 113 sein können. Zwischen den Temperaturausgleichschichten 13 und 113 kann auch ein aus Metall hergestellter, z.B. mäanderförmiger Kanal ausgestaltet werden, welcher eine sehr gute Wärmeübergabe nach der Flüssigkeit 27 sichert. Im gegebenen Falle können die Temperaturausgleichschichte 13 und 113 auch weglassen werden und die Isolierschichte 15 und 115 als Begrenzungswände des Kanals 47 benutzt werden. Dabei erfolgt der Temperaturausgleich durch die im Kanal 47 strömende Flüssigkeit.

## Ansprüche

1. Heizmittel zur Temperaturregelung von strömenden Medien, enthaltend mindestens zwei erwärmende Seitenwände und zwischen denen einen Kanal für das zu erwärmende Medium, wobei jede Seitenwand mindestens ein erwärmendes Widerstandsnetz aus leitungsfähigem Material und mindestens einen Temperaturfühler zur Messung der Temperatur der Seitenwand aufweist, dadurch **gekennzeichnet,** daß das erwärmende Widerstandsnetz aus einem and der dem zu erwärmenden Medium abgewandten Seite einer elektrisch isolierenden Tragplatte (1, 101) ausgebildeten Heizfoliemuster (2,102) gebildet ist, und daß and der dem zu erwärmenden Medium zugewandten Seite der Tragplatte (1,101) ein einen temperaturfühlenden Widerstand bildendes, aus leitungsfähigem Material hergestelltes Fühlfoliemuster (3,103) ausgebildet ist, welches Fühlfoliemuster (3,103) mit einer Isolierschicht (15,115) bedeckt ist.

2. Heizmittel nach Anspruch 1, dadurch gekennzeichnet, daß an der Isolierschicht (15,115) eine aus wärmeleitendem Material hergestellte Temperaturausgleichschicht (13,113) angeordnet ist.

3. Heizmittel nach Anspruch 2, dadurch gekennzeichnet, daß die Temperaturausgleichschicht (13,113) an der mit Fühlfoliemuster (3,103) versehenen Seite der Tragplatte (1,101) mit einem isolierenden Klebstoff befestigt ist, welcher Klebstoff die Isolierschicht (15,115) bildet.

4. Heizmittel nach Anspruch 3, dadurch gekennzeichnet, daß die Temperaturausgleichschicht (13,113) eine Aluminium-oder Kupferplatte mit einer Dicke von kleiner als 2 mm ist, welche mit Kunstharzklebstoff aus die mit Fühlfoliemuster (3, 103) versehene Seite der Tragplatte (1,101) aufgeklebt ist.

5. Heizmittel nach einem der Ansprüche 1 bis 4, dadurch gekennzeichnet, daß das Heizfoliemuster (2,102) mit einer aus elektrisch isolierendem Material hergestellten Schutzschicht (14,114) überzogen ist.

6. Heizmittel nach einem der Ansprüche 1 bis 4, dadurch gekennzeichnet, daß das Heizfoliemuster (2,102) als eine erste Mäanderformation ausgebildet ist, die aus mehreren, an der Tragplatte (1,101) im wesentlichen unter gleichem Abstand angeordneten parallelen Foliestrecken besteht, daß das Fühlfoliemuster (3,103) als wenigstens eine aus im wesentlichen unter gleichem Abstand angeordneten Foliestrecken bestehende zweite Mäanderformation (4,4A) ausgebildet ist, und daß die parallelen Foliestrecken des Heizfoliemusters (2,102) im wesentlichen senkrecht zu den parallelen Foliestrecken des Fühlfoliemusters (3,103) sind.

7. Heizmittel nach Anspruch 6, dadurch gekennzeichnet, daß das Fühlfoliemuster (3,103) als zwei voneinander elektrisch isolierte und kammartig ineinanderreichende Mäanderformationen (4,4A) ausgebildet ist.

8. Heizmittel nach Anspruch 6, dadurch gekennzeichnet, daß an der mit dem Heizfoliemuster (2,102) versehenen Seite der Tragplatte (1,101) neben dem Heizfoliemuster (2,102) ein davon elektrisch isoliertes, temperaturfühlendes weiteres Fühlfoliemuster ausgebildet ist, welches weitere Fühlfoliemuster als eine in die erste Mäanderformation des Heizfoliemusters (2,102) kammartig eingreifende dritte Mäanderformation ausgebildet ist.

9. Heizmittel nach einem der Ansprüche 1 bis 4, dadurch gekennzeichnet, daß die Heiz- und Fühlfoliemuster (2,102; 3,103) durch selektive Ätzung einer. beidseitig folierten Tragplatte (1,101) hergestellt sind.

10. Heizmittel nach einem der Ansprüche 1 bis 4, dadurch gekennzeichnet, daß die Heiz- und Fühlfoliemuster (2,102; 3,103) auf die Tragplatte (1,101) durch Vakuumverdampfung aufgetragen sind.

11. Heizmittel nach einem der Ansprüche 1 bis 4, dadurch gekennzeichnet, daß der Kanal (47) zwischen den zwei Seitenwänden (28,128) als ein für das zu erwärmende Medium mit einer Einflußöffnung (45) und einer Ausflußöffnung (46) versehener flacher Beutel (26) ausgebildet ist.

Fig.4

Fig.1

Fig. 2

Fig. 3

Fig. 5

Fig. 6

Fig. 7

EP 0 380 140 A2

Fig. 8

Fig. 9

EP 0 380 140 A2

Fig. 10

Fig. 11

Fig. 12

EP 0 380 140 A2

Fig.13

EP 0 380 140 A2